(19) Europäisches Patentamt
European Patent Office
Office européen des brevets

(11) Publication number: **0 099 567**
**B1**

(12) # EUROPEAN PATENT SPECIFICATION

(45) Date of publication of patent specification: **11.10.89**

(51) Int. Cl.$^4$: **C 12 Q 1/00,** G 01 N 33/53

(21) Application number: **83107042.0**

(22) Date of filing: **18.07.83**

(54) **Improved enzyme assay and method.**

(30) Priority: **19.07.82 US 399168**

(43) Date of publication of application:
**01.02.84 Bulletin 84/05**

(45) Publication of the grant of the patent:
**11.10.89 Bulletin 89/41**

(84) Designated Contracting States:
**DE FR GB IT NL**

(56) References cited:
**EP-A-0 034 213**
**GB-A-1 595 101**
**GB-A-2 001 172**
**GB-A-2 023 607**
**US-A-4 134 792**
**US-A-4 230 797**

**CHEMICAL ABSTRACTS, volume 93, no. 15, October 13, 1980, page 343, abstract 145778q (COLUMBUS, OHIO, US) T.T. NGO et al. "Enzyme modulators as tools for the development of homogenous enzyme immunoassays", & Febs. Lett., vol. 116, no. 2, 1980, 285-288**

(73) Proprietor: **COOPERBIOMEDICAL, INC.**
**3145 Porter Drive**
**Palo Alto California 94304 (US)**

(72) Inventor: **Nix, Paul Thomas**
**62 Forest Drive**
**Jackson, N.J. 08527 (US)**
Inventor: **Hsu, Mei-Yung**
**17 Glendale Drive**
**Englishtown, N.J. 07726 (US)**
Inventor: **Rahfield, Richard Edward**
**RD 53 Gail Chambers Road**
**Jackson, N.J. 08527 (US)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 099 567 B1**

## Description

The present invention concerns analytical enzyme assays and is particularly directed to improvements in the determination of enzymatic rates in analytical assays using specific enzyme modulators.

The most useful property of that category of proteins known as enzymes is their power to catalyze particular chemical reactions. With very few exceptions, the presence and specific activity of enzymes are measured by the occurrence of specific reactions which convert substrate into end products. Typically, the quantity of active enzyme present in the reaction system is estimated from the speed of reaction under conditions such that the rate of reaction is limited by the concentration of enzyme. The measurement of the reaction velocity, regardless of the type of enzyme or the particular reaction system in which it is used, remains the most critical part of analytical assays. Inaccurate measurement of the enzymatic rate of reaction always reduces the accuracy and precision of the assay.

A valuable addition to enzymatic analytical assays is the use of active modulators which can alter or modify the characteristic activity of the enzyme, most notably by a change in the speed of conversion of the substrate into product — the reaction velocity. Such active enzyme modulators include accelerators, agents able to increase the velocity of the enzyme reaction, and decelerators, which reduce the velocity of the reaction. The category of decelerators is very large and includes enzyme inhibitors which in turn may be subdivided into the classes of reversible inhibitors, irreversible inhibitors, and allosteric effectors. Assays for the quantitation of specific enzymatic inhibitors are well known in the art and are exemplified by the publication of Brown, Johnson, Witte and Feld, "Enzymatic Inhibition Assay For Methotrexate With A Discrete Analyzer, the ABA—100", *Clinical Chemistry, 26*:355—338 (1980). Specific enzyme inhibitors from each of these classes have also been used in more sophisticated analytical systems in the form of ligand conjugated inhibits as exemplified by the immunoassays described in the U.S. Patent No. 4,134,792; U.K. Patent No. 1,595,101; and U.S. Patent Nos. 4,273,866 and 3,935,074. Other commercially available analytical systems which are also dependent upon accurate measurement of changes in the enzymatic rate are illustrated by the EMIT System as described by Rosenthal *et al. Clinical Chemistry, 22*:1899 (1976).

US—A—4,134,792 and GB—A—1,595,101 describe binding assays employing an enzyme modulator (inhibitor) which can be conjugated to a ligand.

US—A—4,230,797 describes a heterogeneous specific binding assay using a coenzyme as labeling substance. It further discloses regenerating systems for cofactors and substrates.

EP—A—0,034,213 describes diagnostic enzymatic assay systems containing substrate, enzyme and coenzyme which are stabilized by the addition of means for regenerating the coenzyme.

The kinetics of enzyme reactions, particularly the measurements of reaction rates and the effects of different kinds of active enzyme modulators upon the reaction rate are well described in theory and practice by the texts of Lehninger, *Biochemistry,* (2nd ed), Worth Publishers, Inc. 1975 and Dixon and Webb, *Enzymes,* Academic Press, 1979. Because a thorough comprehension of enzyme kinetics is crucial to a full and complete understanding of the present invention (but a tedious repetitious recital of the state of the art will be avoided), the entire text of the above references is expressly incorporated by reference herein. These texts shall serve as a basis and background against which to identify and place in perspective the valuable and important advances to be described hereinafter.

It is well recognized that a general characteristic of all enzymatic reactions is that the enzymatic rate at which substrate is converted into product will decrease over time. Various reasons attributable to cause this decreasing rate. These include: the buildup of reaction products which acts to reduce the rate of catalysis; the reverse reaction may become more important as the concentration of reaction product increases; the enzyme itself may undergo some inactivation due to a change of temperature or pH. The most likely cause of reduced enzymatic reaction velocity, however, is a decrease in the degree of saturation of the enzyme with substrate because of a corresponding reduction of substrate concentration as the reaction proceeds to completion. For these reasons, the usual approach for determining enzyme concentration utilizes measurement of only the initial velocity of the enzyme. It is only during this initial period in the reaction that the various causes for rate changes identified above have not yet had time to influence the enzyme reaction and thus it is only during this initial period that the reaction is rate limited by the enzyme concentration alone. Moreover, to determine the initial rate of an enzyme, one need only obtain empirical data showing the change in concentration of either the reactants or the products during the early stages of the reaction. If one collects such initial rate data for varying concentrations of an enzyme in an assay system, and then plots it graphically as a standard curve to show observed rate vs. enzyme concentration, one will obtain a straight line if the reaction rates are truly proportional to the enzyme concentration. In fact, however, such linearity exists only for low concentrations of enzyme and solely for short periods of time because of the changes in the composition of the assay media caused by the very occurrence of the reaction itself or due to the kinetics of the enzyme.

It is generally acknowledged, therefore, that standard curves based on initial rates are a true measure of enzyme concentration only so long as strict time and substrate limits are adhered to. Frequently, however, analytical enzyme assays and immunoassays employing an inhibitor or a ligand conjugated to an inhibitor often neglect and exceed both these time and substrate limitations. As a result, many such enzymatic assays are neither accurate nor precise. Nevertheless, such errors in the determination of the enzymatic reaction rate over time, can be eliminated or substantially reduced by the present invention.

A method for determining the activity of an enzyme in an unknown test sample is provided said activity being a function of the quantity of active enzyme modulator present in the sample, comprising the steps of combining as an assay system an enzyme whose activity is altered in the presence of the active enzyme modulator, a substrate suitable for conversion by said enzyme, and a cofactor required by said enzyme for conversion of said substrate into reaction product; regenerating said substrate and/or regenerating said cofactor; and measuring the activity of said enzyme. In addition, the means for such regeneration may form at least one other product in addition to the reactant as a result of the regeneration, the formation of this other product serving as a qualitative and quantitative measure of the active enzyme modulator present in the test sample. The means for regenerating the reactant(s) stabilizes the enzyme velocity such that the reaction rate becomes essentially constant over substantial periods of time.

The detailed description of the present invention may be more completely understood when taken in conjunction with the following figures in which:

Fig. 1 is a graph illustrating the decrease in enzyme rate as a function of time for varying concentrations of the enzyme, dihydrofolate reductase;

Fig. 2 is a graph illustrating the effect of time on the linearity of the enzymatic assay plotted in Fig. 1;

Fig. 3 is a graph illustrating the enzymatic rates of dihydrofolate reductase as a function of time when cofactor regeneration means are introduced into the assay;

Fig. 4 is a graph illustrating the improved linearity in the dihydrofolate reductase assay using the data of Fig. 3;

Fig. 5 is a graph comparing the rates of enzyme reaction while varying the concentration of inhibitor as a function of time in the presence and absence of cofactor regeneration means;

Fig. 6 is a graph illustrating the linearity of the dihydrofolate reductase assay for inhibitor using the data of Fig. 5;

Fig. 7 is another graph illustrating the improvement of the linearity of dihydrofolate reductase assay for inhibitor when cofactor regeneration is present using the data of Fig. 5;

Fig. 8 is a graph illustrating the effect of cofactor regeneration means combined with substrate regeneration means in the dihydrofolate reductase assay;

Fig. 9 is a graph illustrating the effect of time on the linearity of the dihydrofolate reductase assay using only substrate regeneration means from the data of Fig. 8;

Fig. 10 is a graph illustrating the effect of time on the linearity of the enzyme assay with cofactor regeneration means and substrate regeneration means using the data of Fig. 8.

Within the context of this disclosure, the following definitions shall be employed:

"Enzyme" is any protein having at least one catalytic site which functions to convert reactant into product.

"Reactant" is any chemical species undergoing chemical reaction.

"Product" is any chemical entity which was formed via enzymatic reaction.

"Substrate" is the chemical entity upon which an enzyme exerts catalytic action.

"Cofactor" is the chemical entity comprising a metal ion or an organic molecule which is rquired for catalytic activity by an enzyme. If a required cofactor is removed from the enzyme, the enzyme becomes catalytically inactive.

"Coenzyme" is an organic molecular cofactor which blinds to a specific site on the structure of the enzyme. Coenzymes act as intermediate carriers of functional groups, specific atoms or electrons that are transferred during the overall enzymatic reaction.

"Ligand" is any ion or molecule which reacts with another molecule to form a complex. Ligands frequently are macromolecules such as antigens, antibodies, or therapeutic drugs.

"First-order reactions" are those enzymatic reactions which proceed at rates directly proportional to the concentration of one reactant.

"Second-order reactions" are those enzymatic reactions in which the rate is proportional to the product of the concentrations of two reactants (or to the second power of a single reactant).

"Zero-order reactions" are those enzymatic reactions which proceed at a rate varying with factors other than the concentration of the reactants. These are usually proportional to the enzyme concentration.

"Active Enzyme" is that portion of the total quantity of enzyme present which is catalytically functional and is able to convert substrate into product.

"Active Enzyme Modulator" is an entity which binds with an enzyme such that the extent or the mode of enzyme activity is modified. This modification may result in an acceleration or deceleration of enzyme velocity, enzyme activation, a change of substrate specificity or any other alteration of characteristics which is detectable by a change in enzyme activity or change in the mode of enzyme activity. Active enzyme modulators may vary in size from a small molecule to a macromolecule. Their interacton with an enzyme can be either reversible or irreversible depending upon the strength of the intermolecular association: weak intermolecular associations rapidly equilibrate while strong intermolecular associations do not equilibrate or show slow equilibration.

"Enzyme Inhibitor" is an active enzyme modulator which on interaction with an enzyme will decrease its catalytic activity. The mode of action of the inhibitor may be competitive, noncompetitive, uncompetitive, allosteric or any combination of these modes.

"Recycling System" is any chemical reaction or series of chemical reactions which replenishes the

supply of cofactor to an enzymatic reaction solely for the purpose of driving a specific enzymatic reaction to completion in order to maximize the quantity of substrate being converted into reaction product.

"Regeneration Means" are those necessary reactants, enzymes, catalysts, and chemical reaction or series of chemical reactions by which at least one of the products formed by the enzyme reaction is regenerated as a reactant in a form suitable for conversion again into product by the enzyme. Characteristically, the regeneration means involve a decrease in the kinetic order of the reaction and provide rates of reaction proportional to the enzyme concentration.

The present invention is an improvement in analytical assay methods which measure the reaction velocity of an enzyme to determine the presence and concentration of the enzyme in a test sample of unknown composition. Typically, the analytical assay method uses a substrate, a cofactor and an active enzyme whose catalytic conversion of substrate into product is followed to determine the amount of active enzyme present. After the enzymatic reaction rate is accurately measured, this rate, in conjunction with a standard curve plotting rate vs. enzyme concentration, will provide an accurate and precise determination of the amount of active enzyme present in the test sample. In some instances, the amount of active enzyme, unaltered by the presence and influence of an active enzyme modulator, is the only information sought. In diagnostic and clinical analytical assay systems, however, the change in the amount of active enzyme which remains catalytically functional in the presence of an unknown quantity of an active enzyme modulator is sought to determine the concentration of modulator, such modulators often being enzyme inhibitors.

A well-known example of an active enzyme modulator is the reversible enzyme inhibitor, methotrexate. The need to accurately and quantitatively monitor its presence has become particularly necessary because of its prevalent use in cancer treatment. Methotrexate (or its analog aminopterin) is also useful when it is conjugated to a ligand (typically an antigen, antibody or therapeutic drug) to determine the total concentration of another corresponding ligand in the test sample. Such assay methods have been identified earlier and need not be recited in detail again. For this reason, the preferred embodiments of the present invention utilize one model analytical assay method employing the enzyme dihydrofolate reductase (hereinafter DHFR) and the reversible enzyme inhibitor, methotrexate. The preferred embodiments accurately measure the reaction rates for DHFR over greater periods of time than was possible before, and, through the obtained empirical data, precisely determine the presence and quantity of any different kinds of compositions able to bind to a specific ligand.

The dihydrofolate reductase (hereinafter DHFR) analytical assay system comprises an aqueous solution containing DHFR, dihydrofolate (hereinafter $FH_2$) the reduced cofactor nicotinamide adenine dinucleotide phosphate (hereinafter NADPH) and the various salts and buffers as described herein. The DHFR catalyzes the reduction of $FH_2$ to tetrahydrofolate (hereinafter $FH_4$) with the concomitant oxidation of NADPH to nicotinamide adenine dinucleotide phosphate (hereinafter NADP) as shown in Reaction Scheme 1.

Reaction Scheme 1

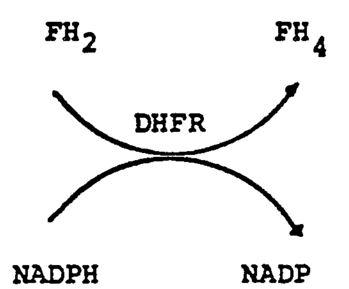


It will be appreciated by those ordinarily skilled in the art that both $FH_2$ and NADPH individually are well known as cofactors which are commonly employed in many other enzymatic analytical assay systems. However, within the DHFR assay model system one may arbitrarily label either one of these reactants as the substrate and the other as the cofactor. Each reactant can be regenerated independently of the other within the DHFR analytical model system. In other enzymatic analytical assays, however, it is anticipated that these substances in either their oxidized or reduced forms will function primarily as cofactors.

The velocity at which the DHFR reaction shown in Reaction Scheme 1 proceeds is measured by observing (and recording) the decrease of light absorbance of the reaction solution at 340 nanometers (hereinafter nm). Both $FH_2$ and NADPH absorb light at 340 nm almost equally at test concentrations. The reaction products, $FH_4$ and NADP, do not absorb light at this wavelength.

The DHFR assay is rate limited by the quantity of catalytically active DHFR present in the assay system. The rate of reaction is determined by measuring the initial rate of substrate conversion as indicated by the decrease in light absorbance at 340 nm over time, the rate of decrease of absorbance being proportional to the active DHFR concentration in the assay. For an accurate determination of the reaction velocity using this

procedure, it is necessary that both the $FH_2$ and the NADPH initially be present and continuously remain in sufficiently high concentrations that the DHFR is saturated — that is, all the catalytic sites present on the enzyme be fully occupied with substrate at all times during the reaction. However, since both substrate and cofactor absorb light at 340 nm, there are practical limits as to how much of these reactants may be used in the assay. Specifically, because of the dual requirement that the reactants show a high absorbance at 340 nm and yet remain at saturation concentrations in proportion to the quantity of DHFR throughout the assay only a narrow range of DHFR concentrations can be used and accurately measured.

To demonstrate the effects of a failure to comply with these requirements, the reagent formulation of Brown *et al* (Clin. Chem. *26*:355, 1980) was used in our assay. The concentrations of the components comprising the reagent formulation are given in table 1.

Table 1

| Reagent Component | Concentration |
|---|---|
| $FH_2$ | .085 mM |
| NADPH | .104 mM |
| B-Mercaptoethanol | 14.3 uM |
| Sodium Phosphate | 192 mM |
| Tris buffer | 1.6 mM |
| Bovine Serum Albumin | .2% |
| pH | 6.5 |

The observed enzyme reaction rates were measured at varying times using different concentrations of DHFR, all reagents being held constant. Specifically, one milliliter (hereinafter ml) of Brown's reagent was incubated at 30°C, and the enzyme reaction initiated by addition of 20 µl of DHFR solution at varying concentrations. A unit of DHFR activity is defined as that amount of enzyme catalyzing reduction of one micromole of $FH_2$ per minute. The rate of reaction was monitored by measuring the change in absorbance of light at 340 nm per minute at one minute intervals for 10 minutes. The results are graphically illustrated in Fig. 1.

An analysis of Fig. 1 shows that the Brown reagent formulation produces constant enzyme reaction rates with respect to time only at very low concentrations of DHFR. Fig. 1 further shows Reaction Scheme 1 to be a second order reaction in which the concentrations of both reactants, substrate ($FH_2$) and cofactor (NADPH), are the controlling factors. Thus, in order to obtain even nominally constant reaction rates, the concentrations of the substrate and cofactor must be substantially increased or the quantity of DHFR diluted to lower concentrations.

The data shown in Fig. 1 is plotted more informatively as a family of curves in Fig. 2, each curve representing the observed rates of different enzyme concentrations in milliunits per milliliter (hereinafter mU/ml) in the reaction mixture at varying times. The single solid line is extrapolated from the rate data obtained during the first minute of reaction time for enzyme concentrations below 10 mU/ml. It is apparent that the observed rates for DHFR fall dramatically from the single solid line as the enzyme concentration increases. Moreover, it is clear that some nonlinearity occurs with high DHFR concentrations even when the rates are measured during the first minute and the deviation from linearity at any given concentration of DHFR increases progressively with greater reaction time.

The nonlinearity of reaction velocities in the DHFR assay using the Brown reagent formulation is not obvious to the casual observer since a straight line can usually be fitted to a graphic plot or even marginal data thereby making the results appear reasonably linear. When gross deviations from linearity under initial velocity measurement conditions are observed, they are usually attributed to causes such as variations in procedure, substrate exhaustion and product feedback inhibition and consequently ignored. However, a careful examination of the data shown in Figs. 1 and 2 reveals that the Brown reagent assay is nominally linear only when working with dilute enzyme concentrations. The greatest errors and deviations occur with the higher DHFR concentrations such as are employed in enzyme assays to determine the presence of active enzyme modulators or compositions able to bind with ligand conjugates of active enzyme modulators.

Recognizing the deficiencies in the Brown reagent assay system, one embodiment of the present invention which provides a substantial improvement of the linearity of observed velocities in enzyme assays is shown by the series of reactions in Reaction Scheme 2 below.

Reaction Scheme 2

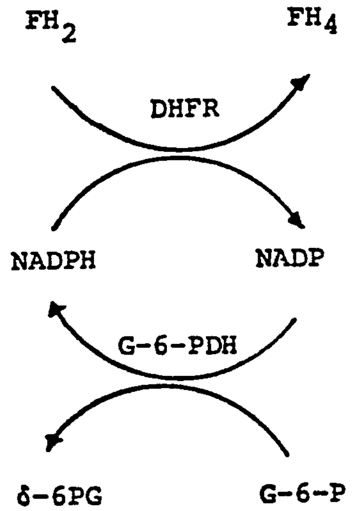


These reactions represent the inclusion of the enzyme glucose-6-phosphate dehydrogenase (hereinafter G6PDH) in the DHFR assay system. The working concentrations of the components are given in Table 2.

Table 2

| Reagent Component | Concentration |
|---|---|
| $FH_2$ | 0.12 mM |
| NADPH | 0.065 mM |
| G-6-P | 3.3 mM |
| G6PDH | 1 U/ml |
| Tris buffer | 114 mM |
| MOPSO buffer | 80 mM |
| Dithioerythritol | 6 mM |
| pH | 7.0 |

The substrate for this additional enzyme is glucose-6-phosphate (hereinafter G6P) which is converted to delta-6 phosphogluconolactone (hereinafter δ-6PG). Together the G6PDH and G-6-P comprise means for regeneration of NADPH for the NADP formed previously by the DHFR enzymatic reaction. This NADPH regeneration means will be more generally referred to as the means for the regeneration of cofactor.

Referring now to Figs. 3 and 4, there appear the graphically plotted effect of the cofactor regeneration means upon the DHFR assay system. The data was obtained in the following manner. One ml of reagent was incubated at 37°C and the enzyme reaction initiated by addition of 20 μl of diluted DHFR solution varying in concentration from 0.0 to 40 mU/ml. The rate of reaction was monitored by measuring the change in light absorbance at 340 nm per minute at one minute intervals for 10 minutes.

It is apparent from the data in Fig. 3 that the NADPH regeneration means provide a substantially constant enzyme velocity even at high concentrations of DHFR over a test interval of 10 minutes. The data in Fig. 3 was replotted in Fig. 4 to show that the cofactor regeneration means produces a marked improvement of linearity even at a 40 mU/ml DHFR concentration. The single solid straight line is obtained by extrapolation of rate data measured during the second minute of reaction. Only after the first four minutes of reaction time do the velocity rates for the DHFR reaction begin to deviate from the initial enzyme velocity rate. It is significant that the severity of these observed deviations are comparatively small when judged against those of the Brown reagent illustrated in Fig. 2. The overall effect of including the cofactor regeneration means in the DHFR assay system, therefore, is to transform the DHFR second order reaction into a first order reaction where the concentration of NADPH no long is a factor controlling or affecting the enzymatic velocity.

Additional complexities arise when an active enzyme modulators such as methotrexate is included in

the DHFR reaction system. Firstly, inhibition of DHFR by methotrexate is not instantaneous; an observable lag period precedes any recordable inhibition of DHFR velocity. Secondly, the inhibitor induced lag time necessitates measurement of the DHFR reaction rates over extended time periods and under dynamic conditions which are drastically different from those as present during the initial stages of the DHFR reaction as previously described. The overall effects of these altered circumstances are demonstrated by a comparison of the linearity of the DHFR reaction rates using ever-increasing concentrations of inhibitor and a constant concentration of DHFR. This comparison was performed with and without the cofactor regeneration means described in Reaction Scheme 2 using the reagent components indicated in Table 3. In all instances the amounts of DHFR was fixed and G6PDH was either present in the quantity indicated or totally absent. The results of the comparison are presented graphically in Figs. 5, 6 and 7.

Table 3

| Reagent Component | Concentration |
|---|---|
| $FH_2$ | .12 mM |
| NADPH | .065 mM |
| G-6-P | 3.3 mM |
| G6PDH | 1 U/ml or none |
| Tris buffer | 114 mM |
| MOPSO buffer | 80 mM |
| Dithioerythritol | 6 mM |
| pH | 7.0 |
| MTX-DPH | 0 to $10^{-6}$M |

Figure 5 graphically compares the changes in observed reaction rates for the DHFR reaction over increasing time with and without the cofactor regenerating means in the presence of different concentrations of an active enzyme inhibitor. One ml of the reagent components of Table 3 were incubated at 37°C and the inhibition reaction initiated by addition of 20 µl of a 1 U/ml solution of DHFR. The rates of reaction were monitored by measuring either the change in absorbance at 340 nm at one minute intervals for 10 minutes. Data was obtained under identical conditions except that one set of assays contained regeneration means for NADPH (shown as solid line curves), while the other (shown as broken line curves) lacked the component G-6-PDH. All of the assay mixtures contained a constant amount of DHFR although the concentration of DHFR inhibitor was varied. The inhibitor used in this study was ligand conjugate of methotrexate, γ-(5,5-diphenylhydantoin-3-aceto hydrazide)-methotrexate (hereafter MTX—DPH) which is used in immunoassays to measure the anticonvulsant drug, diphenylhydantoin (DPH). MTX—DPH was added at test concentrations of (a) 0, (b) 1 × $10^{-8}$M, (c) 5 × $10^{-8}$M, (d) 1 × $10^{-7}$M, (e) 5 × $10^{-7}$M, (f) 1 × $10^{-6}$M.

Generally, Figs. 5—7 reveal that as the inhibitor concentrations increases, the measurable DHFR activity decreases. However, the data in Figure 5 indicate that the DHFR assay method without cofactor regeneration means yields different but continuously decreasing rates for each test concentration of MTX—DPH, the inhibitor. It is apparent therefore, that a single test sample containing but one albeit unknown quantity of this inhibitor would empirically designate a wide variety of different reaction rates for DHFR, each of which would indicate a different concentration of MTX—DPH to be present in the one test sample. In comparison, only when the DHFR assay is combined with the cofactor regenerating means do the observed rates become constant and show zero slope over five minutes times.

Since DHFR activity is expected to be inversely proportional to the concentration of the inhibitor, it is expected that graphical plots of DHFR reaction rates (ΔA/Δt) in relation to the inverse of inhibitor concentration will yield substantially straight lines.

Figures 6 and 7 show the effect of reaction time on the linearity of the DHFR assay in the presence of the ligand conjugated inhibitor, MTX—DPH, with and without cofactors regeneration means for NADPH. The data in Fig. 6 presented are identical to the broken line curves in Fig. 5, but the rates are plotted as an inverse function of the MTX—DPH concentration. Each curve corresponds to the linearity obtained when the rates were measured at the indicated times, 2 minute intervals being used for clarity. The concentrations of MTX—DPH used were: 5 × $10^{-8}$M, 1 × $10^{-7}$M, 5 × $10^{-7}$M, 1 × $10^{-6}$M and 5 × $10^{-6}$M respectively.

It is obvious from Fig. 6 that the DHFR assay *without* cofactor regeneration means provides a family of rate response curves which vary greatly with respect to time. In comparison, use of the cofactor regeneration means with the DHFR assay as shown in Fig. 7, indicates that the degree of DHFR inhibition remains reasonably constant and reproducible over a useful range of inhibitor concentrations. The data in

Figure 7 is taken from the solid line curves in Figure 5, but the rates are again plotted as an inverse function of MTX—DPH concentration.

The most dramatic improvement provided by the cofactor regeneration means in the DHFR assay system occurs in two zones: at relatively high inhibitor concentrations where the reaction rates are low; and at very low inhibitor concentrations over extended time intervals. A major reason why the cofactor regeneration system causes such a dramatic improvement in linearity of the rate response curves in the presence of an active enzyme modulator is that it effectively converts a bimolecular, second-order reaction into a unimolecular, first-order reaction. Again, this is accomplished by maintaining a constant concentration of NADPH above the Km value of DHFR for NADPH, thus simplifying the overall kinetics of the assay system.

The preferred embodiment of the present invention additionally provides substrate regeneration means for the DHFR assay system by a nonenzymatic coupling of a tetrazolium dye, 3-(4,5-Dimethylthiazol-2-yl)-2,5-diphenyl-2H-tetrazolium Bromide (hereinafter MTT) with the DHFR produced $FH_4$ to regenerate $FH_2$. This substrate regeneration means is described in Reaction Scheme 3.

**Reaction Scheme 3**

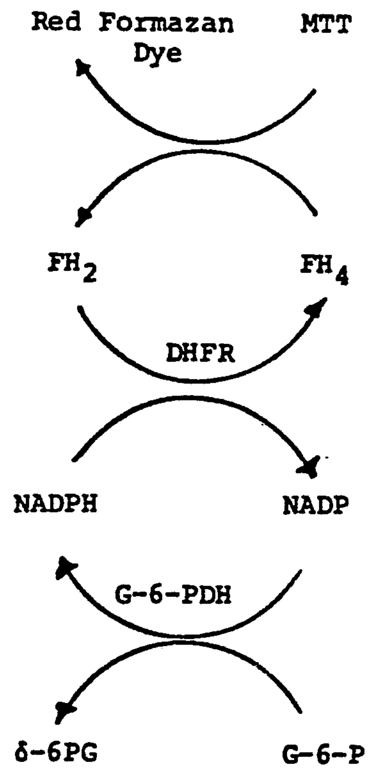


The substrate regeneration means comprises the tetrazolium dye, MTT, which is added to the cofactor regeneration means and the DHFR assay earlier described to convert $FH_4$ into $FH_2$. Kinetically, the MTT combines with the $FH_4$ as it is produced by the DHFR to regenerate $FH_2$ with the concomitant formation of Red Formazan Dye as a by-product. Red Formazan Dye absorbs light only at 568 nm rather than at 340 nm. Accordingly, the regeneration of $FH_2$ may be followed in time by observation of an increasing absorbance at light at 568 nm indicating a mole for mole conversion of $FH_4$ into $FH_2$. When this substrate regeneration means is used in conjunction with the cofactor regeneration means in the DHFR assay system, this combination of regeneration reactions effectively transforms what was originally a second order reaction into a zero order reaction. Concomitantly, there is an additional substantial increase in the linearity of enzyme rate for the DHFR assay.

The additional increase of velocity linearity is graphically shown in Fig. 8 which compares the DHFR assay using the substrate regeneration means alone and in combination with the cofactor regeneration means in the presence of the enzyme inhibitor, MTX—DPH. The reagent components were as indicated in Table 4.

Table 4

| Reagent Compound | Concentration |
|---|---|
| $FH_2$ | .4 mM |
| NADPH | .065 mM |
| MTT | .12 mM |
| G-6-P | 3.3 mM |
| G6PDH | 1 U/ml or none |
| Tris | 114 mM |
| MOPSO | 80 mM |
| DTE | 0 |
| pH | 7.0 |
| MTX-DPH | 0 to $10^{-6}$M |

One ml of the above inhibitor reagents were incubated at 37°C and inhibition reaction initiated by addition of 20 µl of stock solution of DHFR (1 U/ml). The rates of reaction were monitored by measuring the change in absorbance of light at 568 nm at one minute intervals for 10 minutes.

The results of these assays are illustrated graphically in Fig. 8 as a series of curves: the solid line curves represent the effect of both the NADPH and $FH_2$ regenerations means in tandem; the broken line curves portray the effect of the $FH_2$ regeneration means alone, the G6PDH component being absent. The inhibitor, MTX—DPH, is present at test concentrations of (a) 0, (b) $1 \times 10^{-8}$M, (c) $5 \times 10^{-8}$M, (d) $1 \times 10^{-7}$M, (e) $5 \times 10^{-7}$M, and (f) $1 \times 10^{-6}$M. It is readily apparent from Fig. 8 that the DHFR analytical assay using the substrate regeneration means and the cofactor regeneration means in combination provides for linearity of DHFR rates over almost the entire range of inhibitor concentrations tested and throughout the entire test interval of more than ten minutes. Clearly, the added increase in linearity is as dramatic as that shown earlier in Fig. 5 where the cofactor regeneration means and the DHFR assay system alone are compared.

To better demonstrate the effect of the substrate regenerating means of Reaction Scheme 3 in the DHFR assay system, the data in Fig. 8 have been replotted graphically as Figs. 9 and 10.

Fig. 9 shows the effect of time on the linearity of the DHFR assay in the presence of MTX—DPH using only substrate regeneration means for $FH_2$. The data are identical to that of the broken line curves in Fig. 8, but the rates are plotted as a function of inverse inhibitor concentration. Each curve in Fig. 9 corresponds to the linearity obtained when the rates were measured at the indicated times. The concentrations of MTX—DPH actually used were: $5 \times 10^{-8}$M, $1 \times 10^{-7}$M, $5 \times 10^{-7}$M, $1 \times 10^{-6}$M, and $5 \times 10^{-6}$m respectively.

Fig. 10 shows the additional improved linearity of the DHFR-inhibitor assay obtained when both regenerations means for $FH_2$ and NADPH are present. The data are identical to the solid line curves in Fig. 8, but the rates are again plotted as a function of inverse MTX—DPH concentration. Measurement times and inhibitor concentrations are identical to those in Fig. 9.

As is obvious from the comparison between Figs. 9 and 10, the variances of linearity in the inhibited DHFR assay system are greatly diminished and the accuracy of empirical determinations improved when both the substrate regeneration means and the cofactor regeneration means are employed. The deviations from linearity shown by the data in Fig. 9 are believed to be caused by the exhaustion of the cofactor, NADPH, in the DHFR system in the presence of a saturation concentration of $FH_2$, the substrate. This data may be properly compared with that provided in Fig. 6 illustrating the effects of an exhaustion of substrate, $FH_2$, in the presence of a saturation concentration of NADPH, the cofactor, under similar test conditions. It is apparent that the degree of deviation from rate linearity is *not* identical. The differing effects are believed to be caused by the difference in binding affinity, as measured by Km, between the enzyme DHFR and the substrate, $FH_2$, compared to the binding affinity between DHFR for the cofactor NADPH. The differences in the respective binding affinities and the data of Figs. 6 and 9 demonstrate conclusively that the enzyme DHFR binds the $FH_2$ more strongly than the NADPH in the assay system. This observation becomes particularly useful under those test circumstances where the investigator must choose between using either the cofactor regeneration means or the substrate regeneration means alone to improve linearity of reaction velocity.

Although the preferred embodiments of the present invention have been directed to using the DHFR analytical assay system, utilization of means for the regeneration of cofactors and/or substrate will provide a substantial improvement of rate linearity in all enzyme assay systems generally. More uniform and consistent rates of enzyme catalysis will always be obtained using such regeneration means regardless of the enzyme system employed. Most suitable for regeneration are the relatively small number of cofactors which consistently reappear as a requisite component. Cofactor regenerations means are thus the

preferred regeneration means. A noncomprehensive list of cofactors which can be regenerated as part of many other enzyme systems with a resulting increase in rate linearity is given in Table 5.

Table 5

Cofactors in Group-Transferring Enzyme Reactions

| Cofactor | Entity Transferred |
|---|---|
| Nicotinamide adenine dinucleotide | Hydrogen atoms (electrons) |
| Nicotinamide adenine dinucleotide phosphate | Hydrogen atoms (electrons) |
| Flavin Adenine Dinucleotide | Hydrogen atoms (electrons) |
| Flavin mononucleotide | Hydrogen atoms (electrons) |
| Coenzyme | Hydrogen atoms (electrons) |
| Thiamin pyrophosphate | Aldehydes |
| Coenzyme A | Acyl groups |
| Lipoamide | Acyl groups |
| Cobamide coenzymes | Alkyl groups |
| Biocytin | Carbon dioxide |
| Pyridoxal phosphate | Amino groups |
| Tetrahydrofolate coenzymes | Methyl, methylene, formyl or formimino groups |

Similarly, in other analytical enzyme assay systems, the use of regenerating means will convert a second order reaction system into a first order reaction with a corresponding increase in the linearity and reproducibility of measurable rate. Merely representative of specific regenerations means for the regeneration of reactants in enzyme systems other than the DHFR assay, are those listed in Table 6.

Table 6

| Enzyme Modulator | Enzyme | Coenzyme or/ Substrate | Regeneration Means |
|---|---|---|---|
| Flavin adenine dinucleotide | Glucose Oxidase | $O_2/H_2O_2$ | Catalase |
| 5-fluroro-UMP | Thymidylate Synthase | 5, 10-methyl enetetra-hydrofolate/$FH_2$ | Dihydrofolate Reductase, Phosphoribosyl glycinamide formyltransferase |
| Pyridoxal phosphate | Aspartate amino-transferase | 2-oxoglutarate/ glutamate | Glutamate oxidase |
| N-Acetyl glucosamine | Hexokinase | ATP/ADP | Creatine phosphate/ creatine kinase |

**Claims**

1. A method for determining the activity of an enzyme, said activity being a function of the quantity of an active enzyme modulator present in a sample, comprising the steps of:

combining as an assay system an enzyme whose activity is altered in the presence of the active enzyme modulator, a substrate suitable for conversion by said enzyme and a cofactor required by said enzyme for conversion of said substrate into reaction product;

regenerating said cofactor; and

measuring the activity of said enzyme.

2. A method for determining the activity of an enzyme, said activity being a function of the quantity of an active enzyme modulator present in a sample, comprising the steps of:

combining as an assay system an enzyme whose activity is altered in the presence of the active enzyme modulator, a substrate suitable for conversion by said enzyme, and a cofactor required by said enzyme for conversion of said substrate into reaction product;

regenerating said substrate; and

measuring the activity of said enzyme.

3. A method for determining the activity of an enzyme, said activity being a function of the quantity of an active enzyme modulator present in a sample, comprising the steps of:

combining as an assay system an enzyme whose activity is altered in the presence of the active enzyme modulator, a substrate suitable for conversion by said enzyme, and a cofactor required by said enzyme for conversion of said substrate into reaction product;

regenerating said substrate;

regenerating said cofactor; and

measuring the activity of said enzyme.

4. The method for determination as in claim 1 wherein said enzyme activity measuring step comprises determining the change in substrate concentration over time.

5. The method for determination as in claim 2 wherein said enzyme activity measuring step comprises determining the change in cofactor concentration over time.

6. The method for determination as in claim 1 or 3 wherein said regenerating of cofactors step

comprises forming another measurable composition in addition to said cofactor as a result of such regeneration; and

said enzyme activity measuring step comprises determining the change in the concentration of said other composition over time.

7. The method for determination as in claim 1 or 2 wherein said regenerating of substrate step comprises forming another measurable composition in addition to said substrate as a result of such regeneration; and

said enzyme activity measuring step comprises determining the change in concentration of said other composition over time.

8. The method for determination as recited in claim 1, 2 or 3 wherein the modulator comprises an active enzyme modulator conjugated to a ligand.

**Patentansprüche**

1. Verfahren zur Bestimmung der Aktivität eines Enzyms, wobei die Aktivität eine Funktion der Menge eines in einer Probe vorhandenen aktiven Enzym-Modulators ist, umfassend die Stufen:

Zusammenbringen als ein Testsystem eines Enzyms, dessen Aktivität in Gegenwart des aktiven Enzym-Modulators verändert ist, eines für die Umwandlung durch das Enzym geeigneten Substrates und eines vom dem Enzym zur Umwandlung des Substrates in ein Reaktionsprodukt benötigten Cofaktors;

Regenerierung des Cofaktors; und

Messung der Aktivität des Enzyms.

2. Verfahren zur Bestimung der Aktivität eines Enzyms, wobei die Aktivität eine Funktion der Menge eines in einer Probe vorhandenen aktiven Enzym-Modulators ist, umfassend die Stufen:

Zusammenbringen als ein Testsystem eines Enzyms, dessen Aktivität in Gegenwart des aktiven Enzym-Modulators verändert ist, eines für die Umwandlung durch das Enzym geeigneten Substrates und eines vom dem Enzym zur Umwandlung des Substrates in ein Reaktionsprodukt benötigten Cofaktors;

Regenerierung des Substrates;

Messung der Aktivität des Enzyms.

3. Verfahren zur Bestimung der Aktivität eines Enzyms, wobei die Aktivität eine Funktion der Menge eines in einer Probe vorhandenen aktiven Enzym-Modulators ist, umfassend die Stufen:

Zusammenbringen als ein Testsystem eines Enzyms, dessen Aktivität in Gegenwart des aktiven Enzym-Modulators verändert ist, eines für die Umwandlung durch das Enzym geeigneten Substrates und eines vom dem Enzym zur Umwandlung des Substrates in ein Reaktionsprodukt benötigten Cofaktors;

Regenerierung des Substrates;

Regenerierung des Cofaktors; und

Messung der Aktivität des Enzyms.

4. Verfahren zur Bestimmung nach Anspruch 1, in dem die Stufe der Messung der Enzymaktivität die Bestimmung der Änderung der Substratkonzentration über die Zeit umfaßt.

5. Verfahren zur Bestimmung nach Anspruch 1, in dem die Stufe der Messung der Enzymaktivität die Bestimmung der Änderung der Cofaktorkonzentration über die Zeit umfaßt.

6. Verfahren zur Bestimmung nach Anspruch 1 oder 3, in dem die Stufe der Regenerierung von Cofaktoren die Erzeugung einer anderen meßbaren Zusammensetzung zusätzlich zu dem Cofaktor als Ergebnis einer derartigen Regenerierung umfaßt; und

die Stufe der Messung der Enzymaktivität die Bestimmung der Änderung der Konzentration dieser anderen Zusammensetzung über die Zeit umfaßt.

7. Verfahren zur Bestimmung nach Anspruch 1 oder 2, in dem die Stufe der Regenerierung des Substrats die Erzeugung einer anderen meßbaren Zusammensetzung zusätzlich zu dem Substrat als Ergebnis dieser Regenierung umfaßt; und

die Stufe der Messung der Enzymaktivität die Bestimmung der Änderung der Konzentration dieser anderen Zusammensetzung über die Zeit umfaßt.

8. Verfahren zur Bestimmung nach Anspruch 1, 2 oder 3, in der der Modulator einen an einen Liganden konjugierten aktiven Enzymmodulator umfaßt.

**Revendications**

1. Un procédé pour déterminer l'activité d'une enzyme, ladite activité étant fonction de la quantité d'un modulateur actif d'enzyme présente dans un échantillon, comprenant les stades de:

combinaison, comme système d'essai d'une enzyme dont l'activité est modifiée en présence du modulateur actif d'enzyme, d'un sbstrat susceptible d'être transformé par ladite enzyme et d'un cofacteur nécessaire à ladite enzyme pour la conversion dudit substrat en un produit réactionnel;

régénération dudit cofacteur; et

mesure de l'activité de ladite enzyme.

2. Un procédé pour déterminer l'activité d'une enzyme, ladite activité étant fonction de la quantité d'un modulateur actif d'enzyme présente dans un échantillon, comprenant les stades de:

combinaison, comme système d'essai d'une enzyme dont l'activité est modifiée en présence du

modulateur actif d'enzyme, d'un substrat susceptible d'être transformé par ladite enzyme et d'un cofacteur nécessaire à ladite enzyme pour la conversion dudit substrat en un produit réactionnel;

régénération dudit substrat; et

mesure de l'activité de ladite enzyme.

3. Un procédé pour déterminer l'activité d'une enzyme, ladite activité étant fonction de la quantité d'un modulateur actif d'enzyme présente dans un échantillon, comprenant les stades de:

combinaison, comme système d'essai d'une enzyme dont l'activité est modifiée en présence du modulateur actif d'enzyme, d'un sbstrat susceptible d'être transformé par ladite enzyme et d'un cofacteur nécessaire à ladite enzyme pour la conversion dudit substrat en un produit réactionnel;

régénération dudit substrat;

régénération dudit cofacteur; et

mesure de l'activité de ladite enzyme.

4. Le procédé de détermination selon la revendication 1, dans lequel ledit stade de mesure de l'activité enzymatique comprend la détermination du changement de la concentration du substrat au cours du temps.

5. Le procédé de détermination selon la revendication 2, dans lequel ledit stade de mesure de l'activité enzymatique comprend la détermination du changement de la concentration du cofacteur au cours du temps.

6. Le procédé de détermination selon la revendication 1 ou 3, dans lequel ledit stade de régénération du cofacteur comprend la formation d'une autre composition mesurable en plus dudit cofacteur par suit de ladite régénération; et

ledit stade de mesure de l'activité enzymatique comprend la détermination du changement de la concentration de ladite autre composition au cours du temps.

7. Le procédé de détermination selon la revendication 1 ou 2, dans lequel ledit stade de régénération du substrat comprend la formation d'une autre composition mesurable en plus dudit substrat par suite de ladite régénération; et

ledit stade de mesure de l'activité enzymatique comprend la détermination du changement de la concentration de ladite autre composition au cours du temps.

8. Le procédé de détermination défini dans les revendications 1, 2 ou 3, dans lequel le modulateur comprend un modulateur actif d'enzyme conjugué à un ligand.

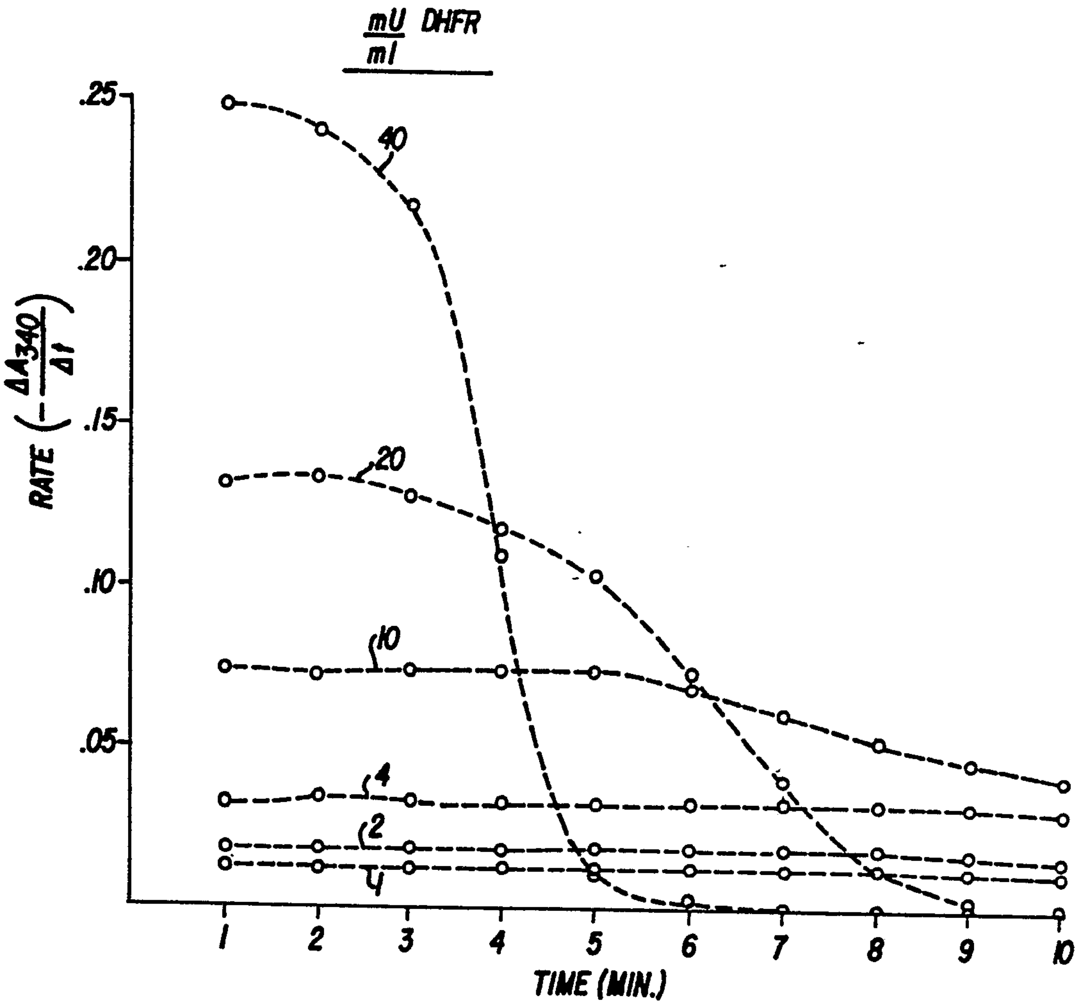
mU/ml DHFR
RATE $\left(-\frac{\Delta A_{340}}{\Delta t}\right)$
.25
.20
.15
.10
.05
40
20
10
4
2
4
1
2
3
4
5
6
7
8
9
10
TIME (MIN.)

FIG. 1

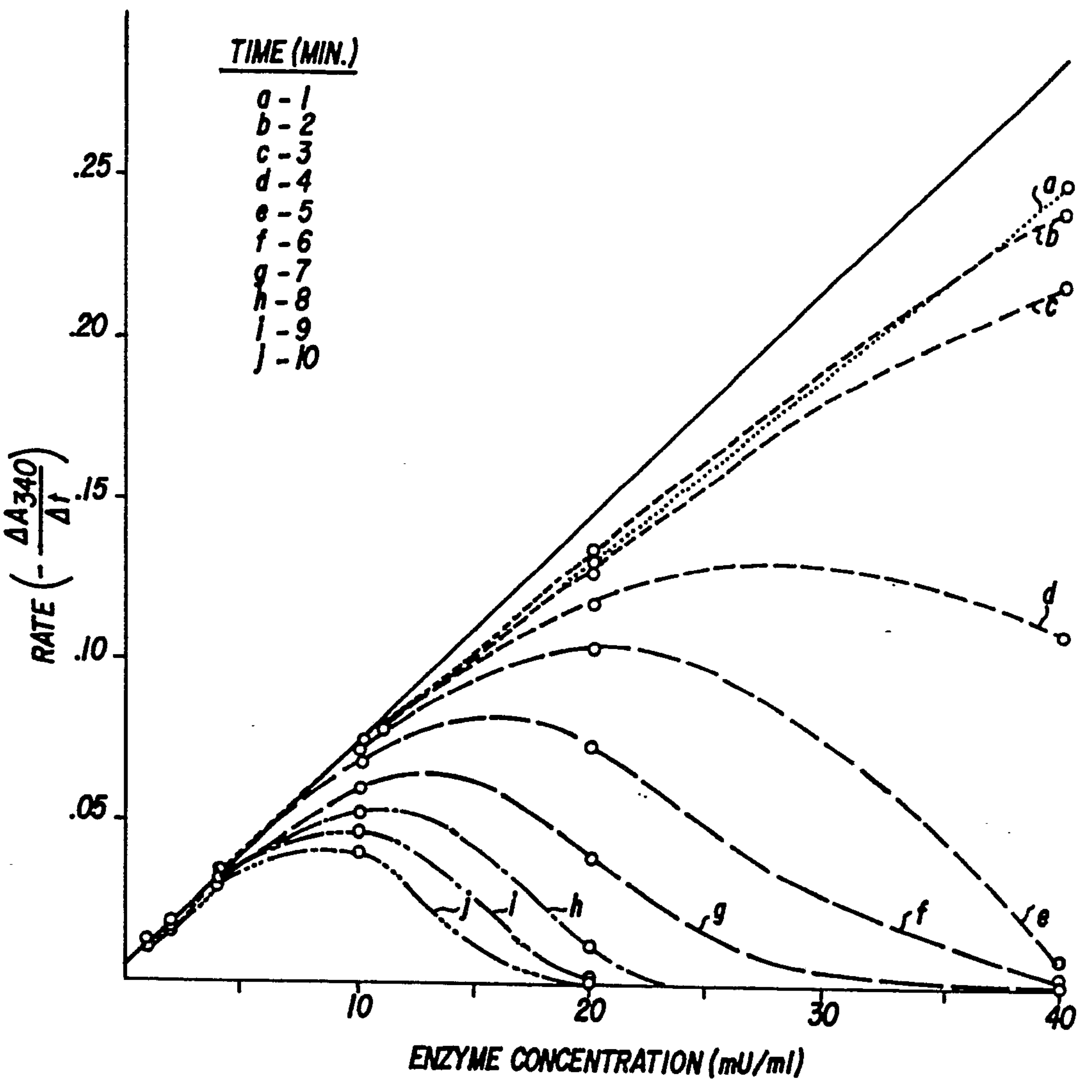
TIME (MIN.)
a - 1
b - 2
c - 3
d - 4
e - 5
f - 6
g - 7
h - 8
i - 9
j - 10
.25
.20
.15
.10
.05
RATE $\left(-\frac{\Delta A_{340}}{\Delta t}\right)$
10
20
30
40
ENZYME CONCENTRATION (mU/ml)
a
b
c
d
e
f
g
h
i
j

FIG. 2

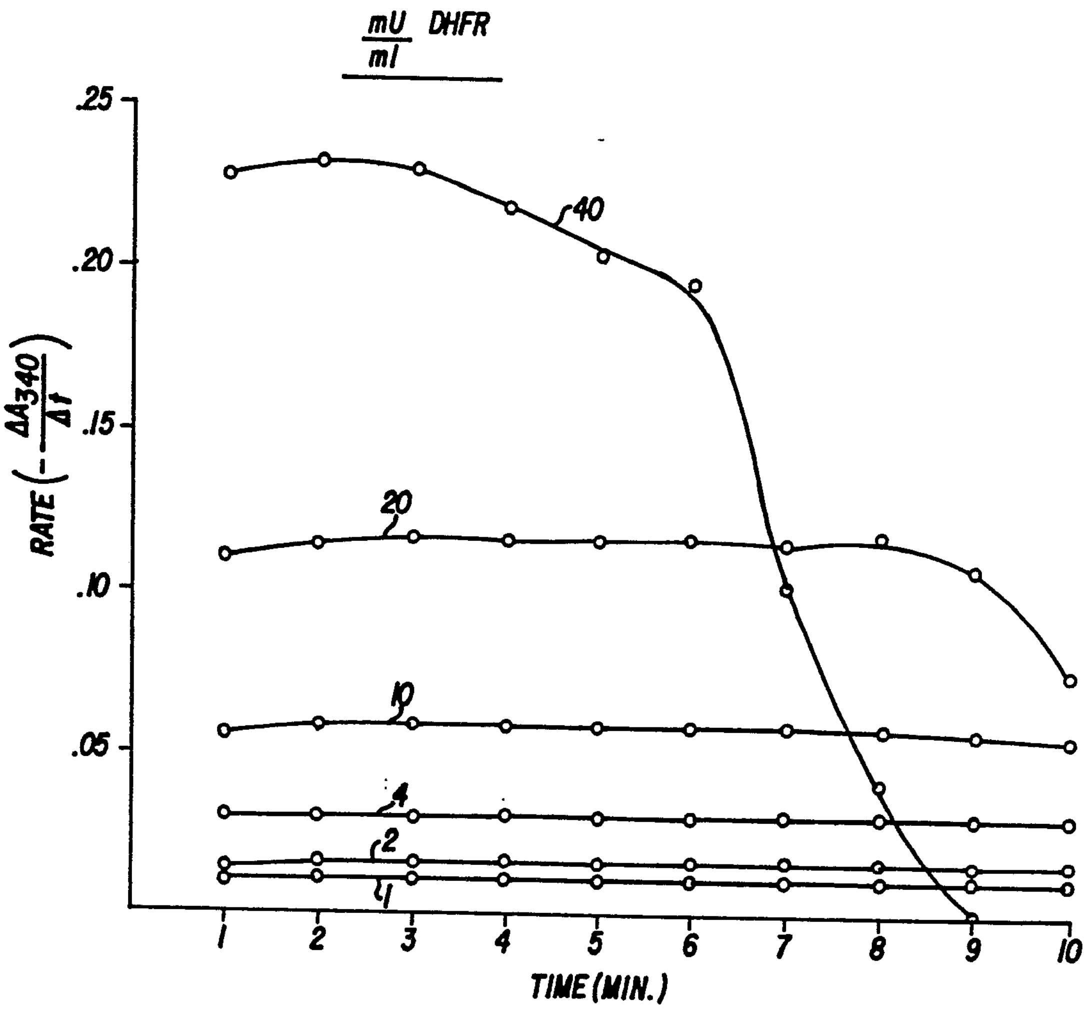
mU/ml DHFR
.25
.20
.15
.10
.05
RATE (-ΔA340/Δt)
40
20
10
4
2
1
1
2
3
4
5
6
7
8
9
10
TIME (MIN.)

FIG. 3

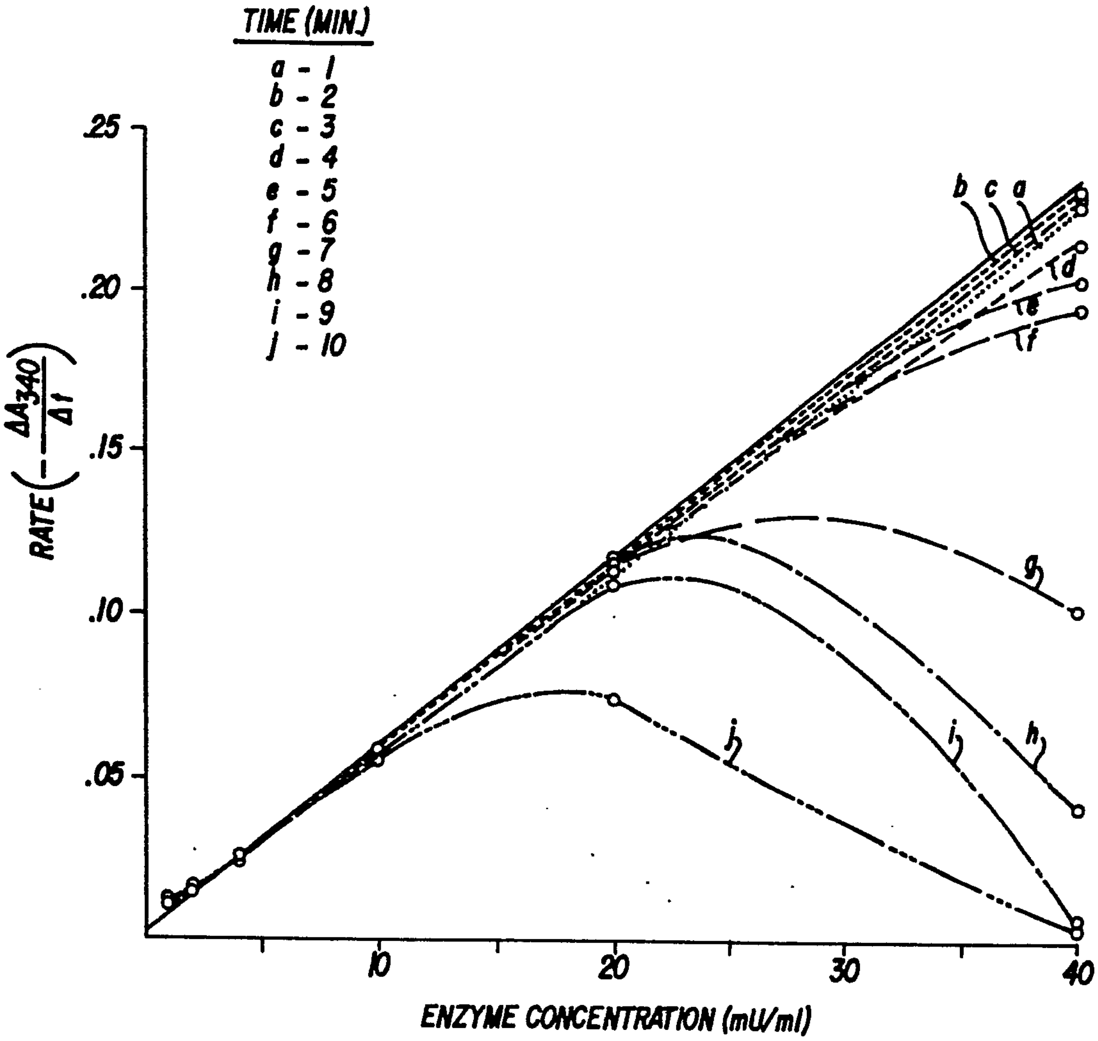
TIME (MIN.)
a - 1
b - 2
c - 3
d - 4
e - 5
f - 6
g - 7
h - 8
i - 9
j - 10
RATE $\left(-\frac{\Delta A_{340}}{\Delta t}\right)$
.25
.20
.15
.10
.05
10
20
30
40
ENZYME CONCENTRATION (mU/ml)
b c a
d
e
f
g
h
i
j

FIG. 4

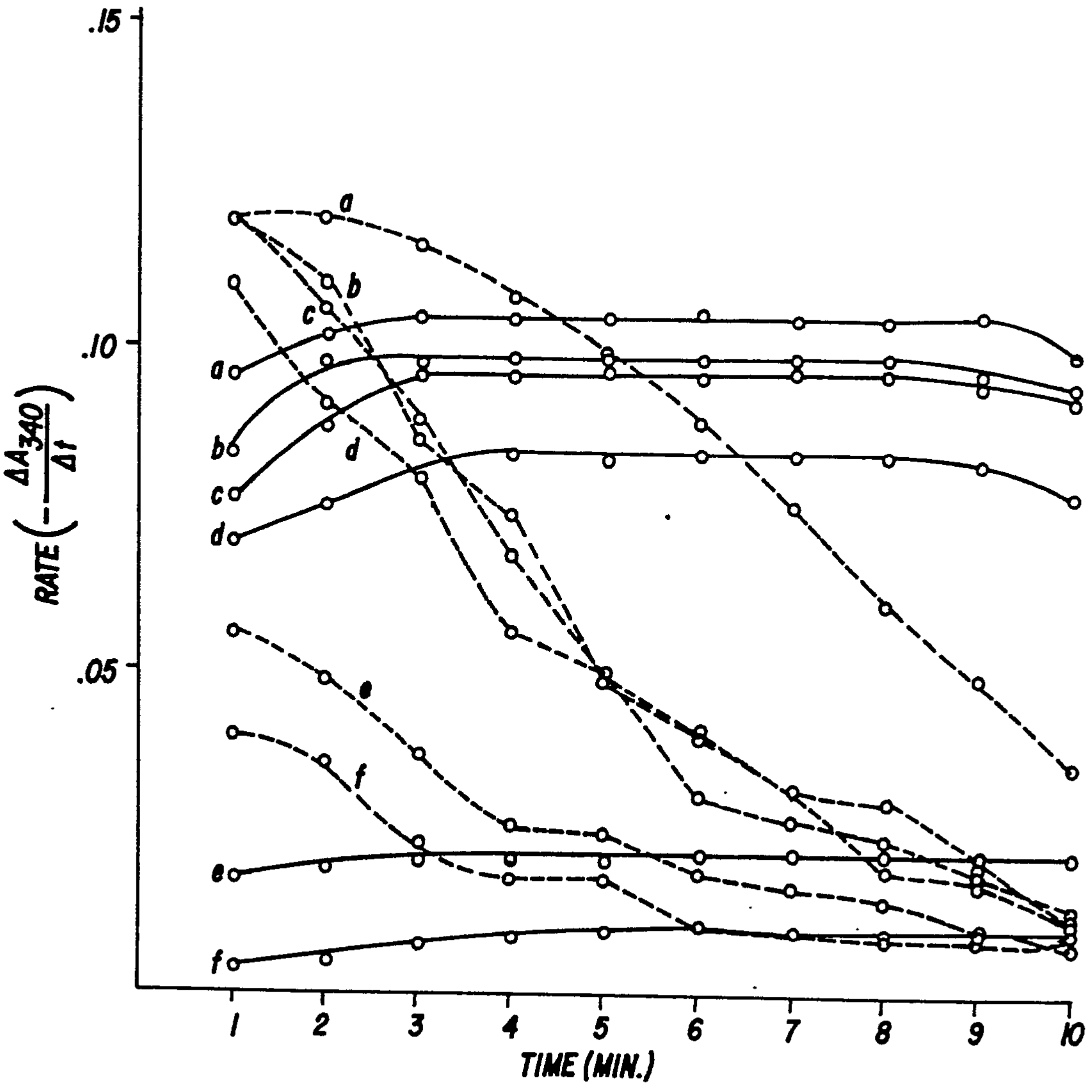


FIG. 5

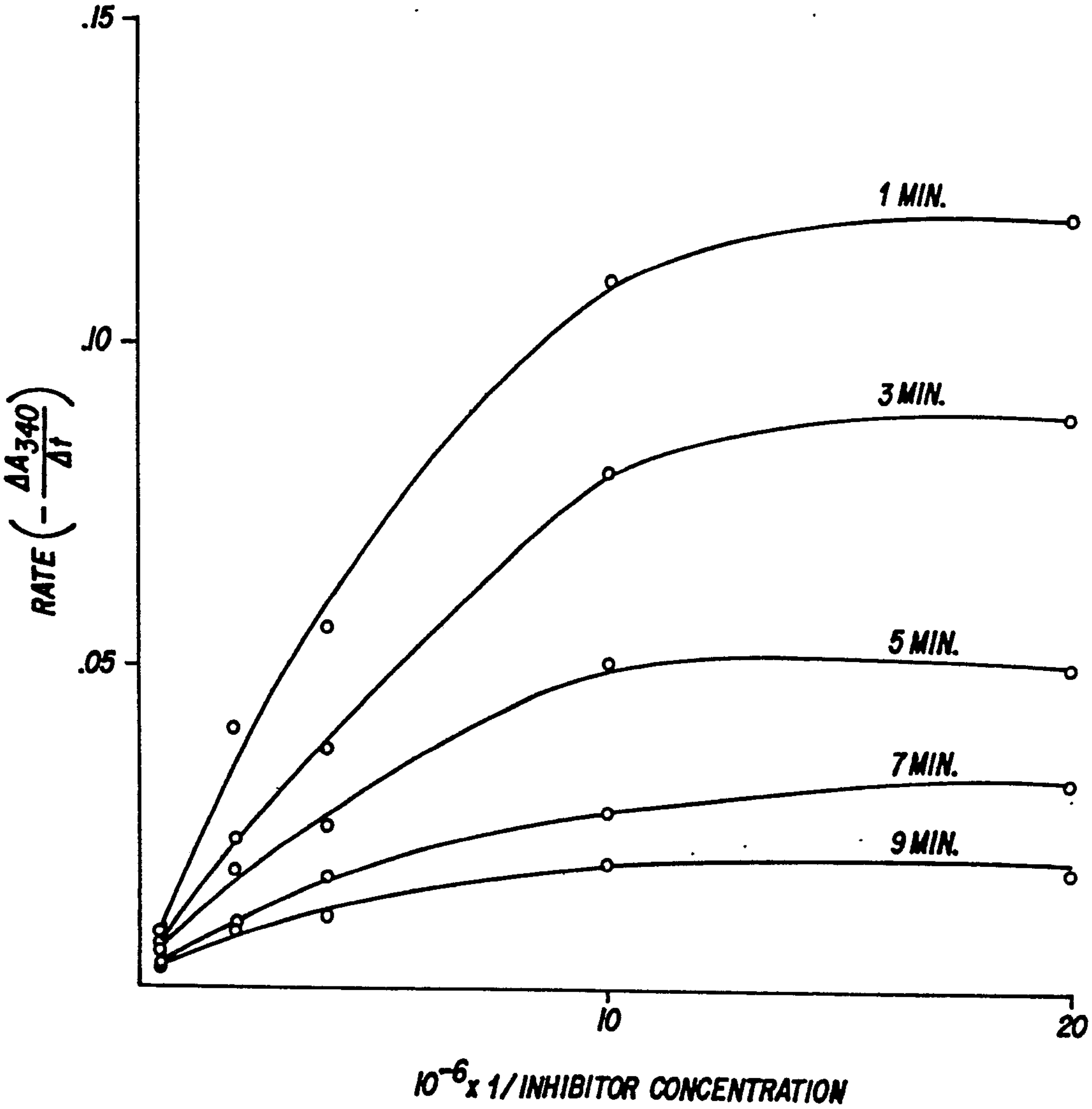
.15
.10
.05
RATE $\left(-\frac{\Delta A_{340}}{\Delta t}\right)$
1 MIN.
3 MIN.
5 MIN.
7 MIN.
9 MIN.
10
20
$10^{-6}$ x 1/INHIBITOR CONCENTRATION

FIG. 6

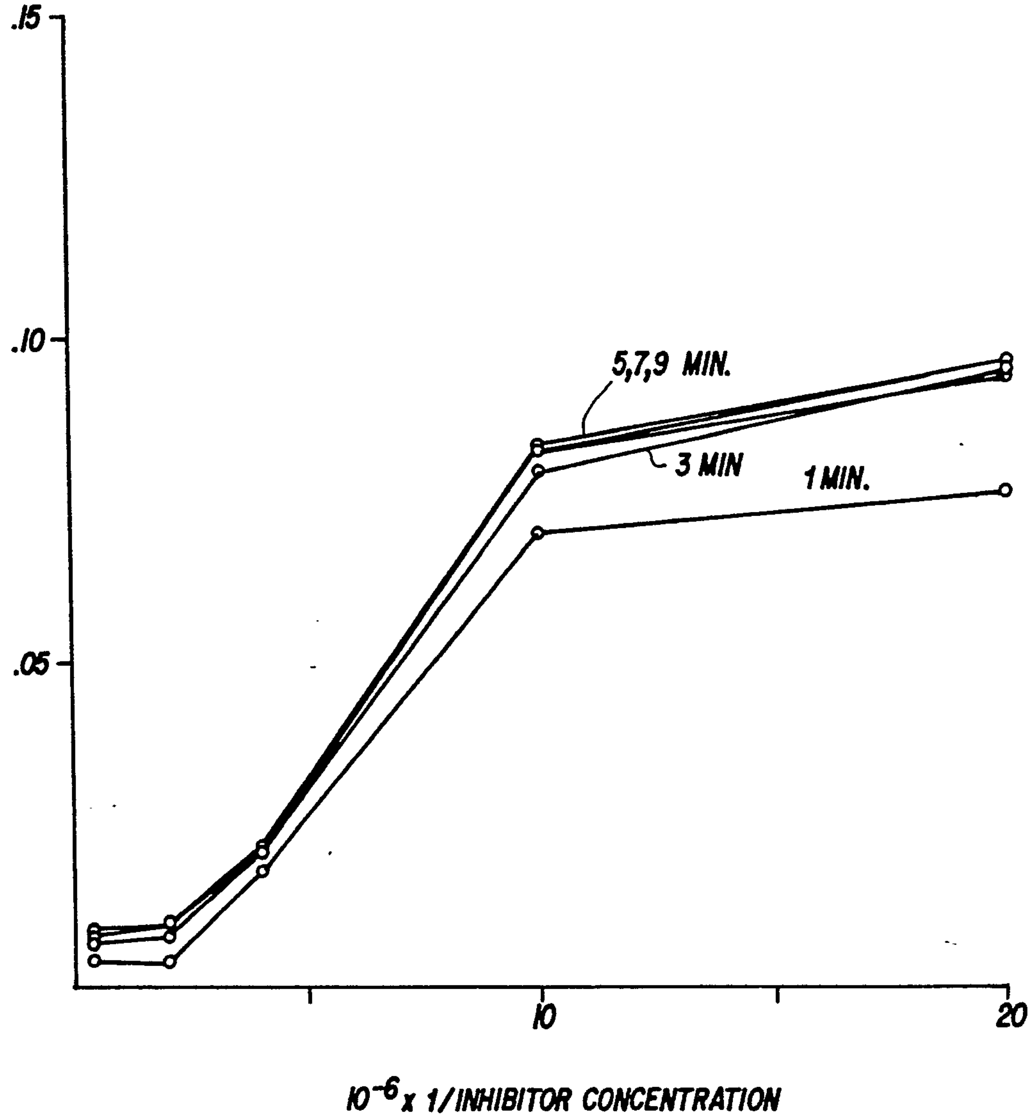
.15
.10
.05
10
20
5,7,9 MIN.
3 MIN
1 MIN.
10^-6 x 1/INHIBITOR CONCENTRATION

FIG. 7

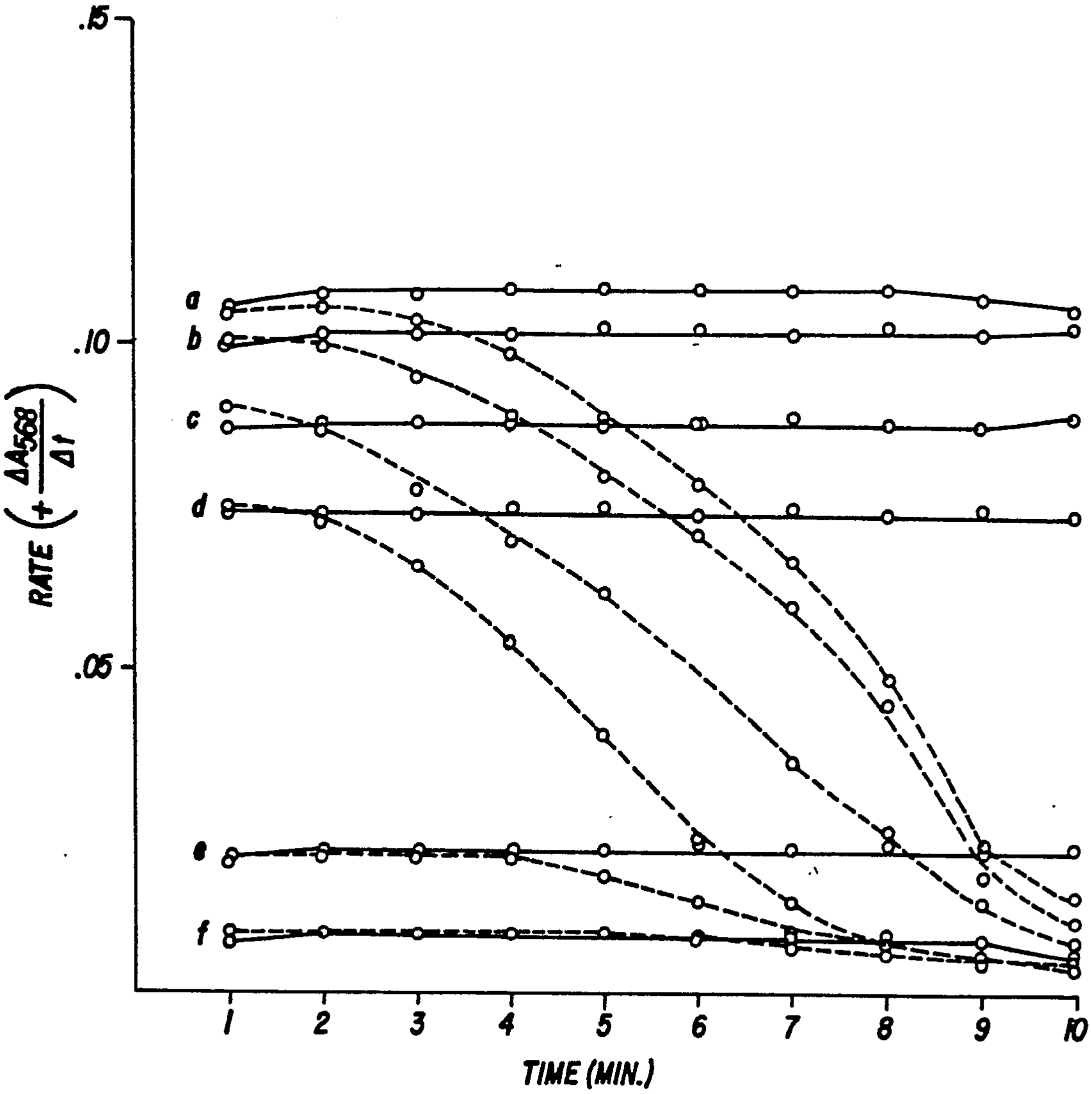


FIG. 8

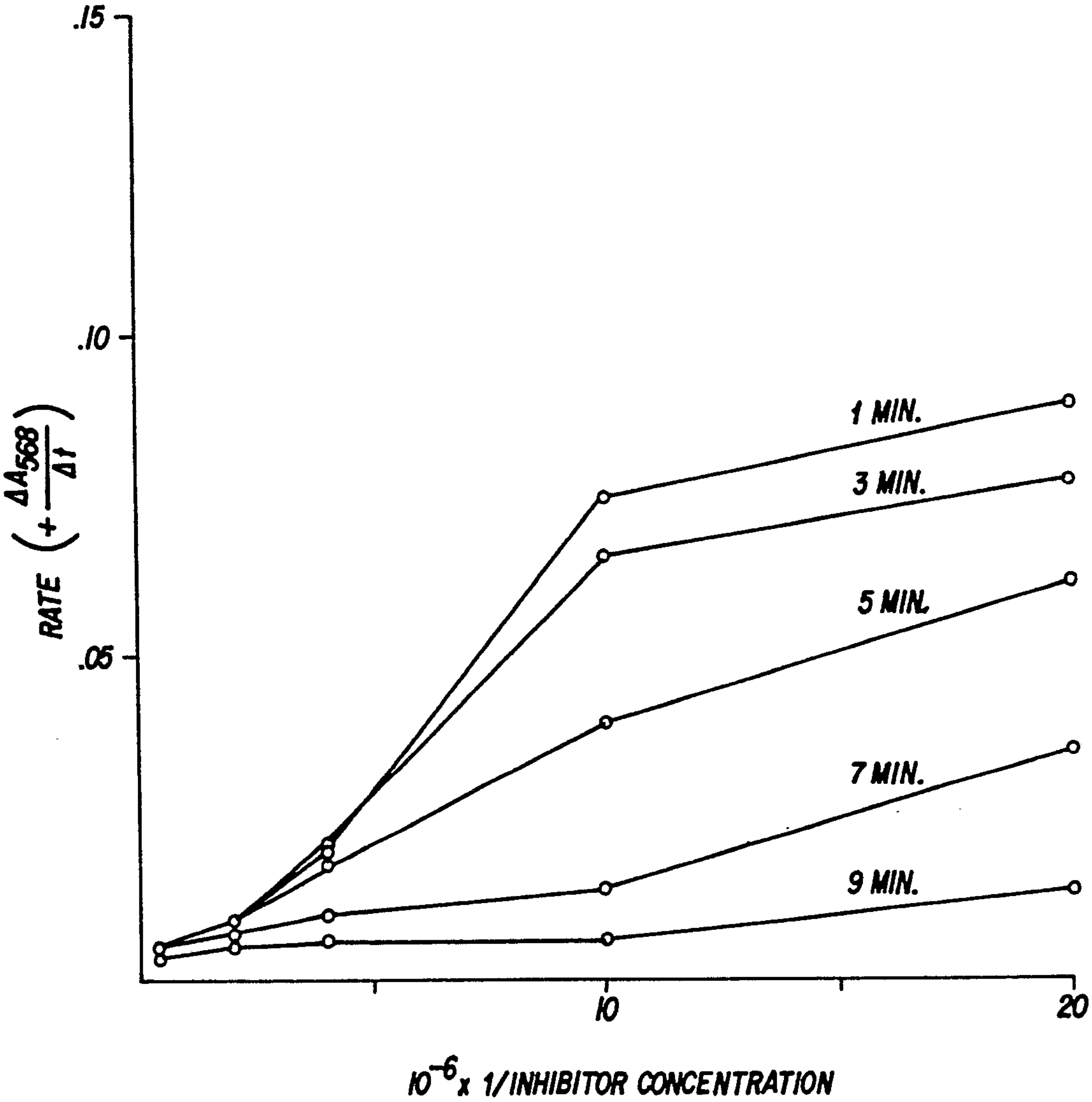
.15
.10
.05
RATE $\left(+\frac{\Delta A_{568}}{\Delta t}\right)$
1 MIN.
3 MIN.
5 MIN.
7 MIN.
9 MIN.
10
20
$10^{-6}$ x 1/INHIBITOR CONCENTRATION

FIG. 9

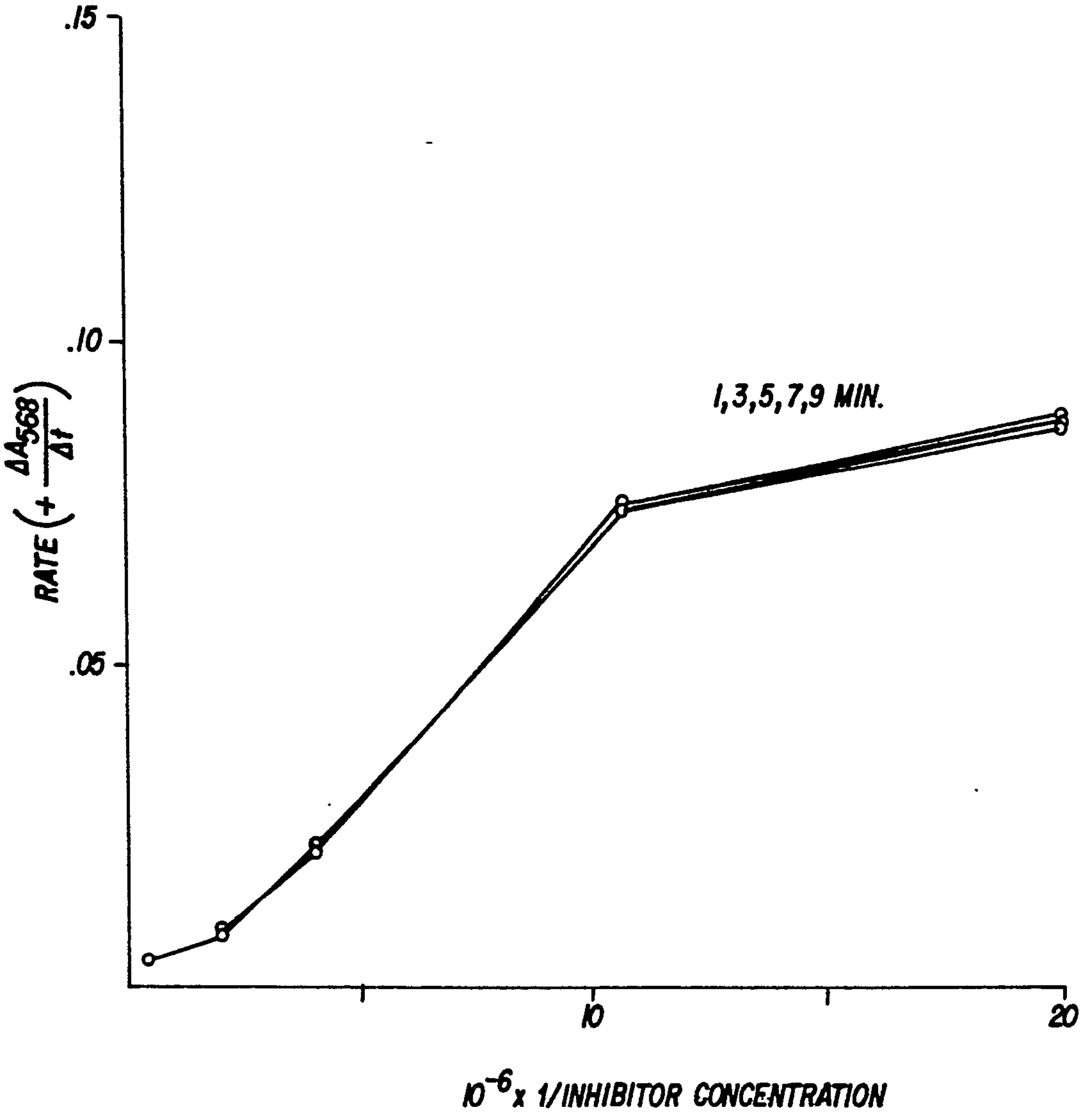
.15
.10
.05
RATE $\left(+\frac{\Delta A_{568}}{\Delta t}\right)$
1,3,5,7,9 MIN.
10
20
$10^{-6}$ x 1/INHIBITOR CONCENTRATION

FIG. 10